# EUROPEAN PATENT APPLICATION

(11) **EP 3 854 412 A1**
(43) Date of publication of application: **28.07.2021**
(21) Application number: 19863513.8
(22) Date of filing: 11.09.2019
(51) Int. Cl.: A61K 38/16, A61P 15/00, A61P 15/06, A61P 15/08, C12N 15/11, C12Q 1/6851, G01N 33/53, G01N 33/569

(54) **AGENT AND COMPOSITION FOR IMPROVING INTRAUTERINE BACTERIAL FLORA, AND METHOD FOR DETERMINING WHETHER INTRAUTERINE BACTERIAL FLORA HAS BEEN IMPROVED OR NORMALIZED**

(30) Priority: 20.09.2018 JP 2018176473
(71) Applicant: NRL Pharma, Inc., Takatsu-ku Kawasaki-shi, Kanagawa 213-0012 (JP); Varinos, Inc., Tokyo 135-0064 (JP)
(72) Inventor: HOSHINO, Tatsuo, Kanagawa 213-0012 (JP); SAKURABA, Yoshiyuki, Tokyo 135-0064 (JP); NAGAI, Yoko, Tokyo 135-0064 (JP); KYONO, Koichi, Tokyo 108-0074 (JP); HASHIMOTO, Tomoko, Tokyo 108-0074 (JP)
(74) Representative: Osha Liang
(86) International application number: PCT/JP2019/035731
(87) International publication number: WO 2020/059600

(57) **Abstract**

The purpose of the present invention is to provide an agent for improving intrauterine bacterial flora, and a method for determining whether intrauterine bacterial flora has been improved or normalized. An aspect of the present invention is an agent for improving intrauterine bacterial flora that contains lactoferrin or a salt thereof as an active ingredient. Additionally provided are: an agent or composition for improving intrauterine flora or treating or preventing diseases caused by the imbalance of intrauterine bacterial flora, the agent or composition containing lactoferrin or a salt thereof; a method for treating or preventing diseases caused by imbalance of intrauterine bacterial flora, the method comprising administrating the agent or composition; and a method for determining whether intrauterine bacterial flora has been improved or normalized.

## Description

### TECHNICAL FIELD

The present invention relates to an agent for improving the intrauterine bacterial flora, a composition for improving the intrauterine bacterial flora, and a method for determining whether the intrauterine bacterial flora has been improved or normalized.

### BACKGROUND ART

In the vaginas of healthy women of reproductive age, bacteria of the genus Lactobacillus abundantly exist, and it has been known that the lactic acid and antibacterial substances produced by Lactobacillus bacteria create an environment in which other pathogenic bacteria, viruses, and the like cannot grow, so that Lactobacillus bacteria play a role in protecting the vagina from infections.

On the other hand, the bacterial environment in the uterus has been considered such that although this environment is spatially connected to the vagina, the environment is sterile due to the presence of a mucosal barrier secreted by the cervical canal (Non-Patent Document 1). In early 2000's, a DNA amplification method for the 16S ribosome RNA (rRNA) gene, which is characteristic of individual bacteria, was established, and it became possible to identify a bacterium from the DNA sequence information of an isolated bacterium. Furthermore, next-generation sequencing technologies that can analyze huge quantities of DNA at once have appeared, and it became possible to investigate the presence of bacteria that are hardly culturable or are in trace amounts, without isolating the bacteria, by collectively analyzing the 16S rRNA genes derived from all bacteria existing in a sample. A bacterial community that is complicatedly composed of a plurality of bacteria is called bacterial flora, microbiota, or flora, and it has become possible to understand which bacteria make up what proportion of the totality. In recent years, it has become clear by an analysis of bacterial flora using this next-generation sequencer that bacterial flora also exists in the uterus. In 2015, Franasiak et al. of Rutgers University in the United States investigated the intrauterine bacterial environment at the time of performing *in vitro* fertilization using a next-generation sequencer and reported that a large quantity of bacteria of the genus Lactobacillus also inhabit the uterus (Non-Patent Document 2). In the same year, Mitchell et al. of the University of Washington also reported that there is vagina-derived bacterial flora in the uterus, and that normal endometrium is inhabited by a large quantity of bacteria of the genus Lactobacillus (Non-Patent Document 3).

The bacterial environment in the reproductive organs is important for reproductive medicine and has been reported to be associated with infertility and recurrent pregnancy loss with recurrent miscarriage, premature birth, or stillbirth. Particularly, the intrauterine flora is very important for understanding the intrauterine environment into which fertilized eggs that have been in vitro fertilized are returned. In 2016, Moreno et al. in Spain investigated the relationship between sterility and the bacterial flora in the vagina and the uterus. As a result, they reported that the intrauterine bacterial flora is very stably maintained during the implantation period, and that a decrease in the bacteria of the genus Lactobacillus correlates to a decrease in the pregnancy rate, the pregnancy continuation rate, and the delivery success rate in *in vitro* fertilized patients (Non-Patent Document 4). Similarly, in 2017, Jia et al. in China investigated the bacterial flora in the female genital tract and reported that a unique bacterial flora is established in the genital tract from the vagina to the fallopian tubes. Bacteria in the vagina and the uterus show a correlation with genital diseases associated with sterility, and a possibility has been suggested that those bacteria may be used as biomarkers for diseases in the future (Non-Patent Document 5).

The inventors of the present invention have established a unique sampling method and a unique analysis method for detecting the intrauterine bacterial flora in which the abundance of bacteria is approximately 1,000 to 10,000 times less than that of the vagina (Non-Patent Document 6). The intrauterine and intravaginal bacterial flora of 7 healthy volunteers, 79 *in vitro* fertilization patients, and 23 general infertility patients was investigated by these methods, and as a result, a divergence between the intravaginal bacterial flora and the intrauterine bacterial flora was recognized in about 30% of the entire test subjects. While the occupancy rate of intrauterine Lactobacillus bacteria was 99.50% on the average in healthy volunteers, the occupancy rate was 63.90% on the average in *in vitro* fertilization patients. In addition, the Lactobacillus occupancy rate in 18 subjects who became pregnant during the study period was 96.45%, and it was revealed that the occupancy rate of intrauterine Lactobacillus bacteria is high in the group of pregnant subjects.

As described above, it has become clear by a new analysis technology that a bacterial flora different from the bacterial flora in the vagina is constituted in the uterus. To date, it has been pointed out that the intrauterine bacterial flora is associated with the pregnancy outcome as well as endometriosis, endometrial cancer, endometritis, and chorioamnionitis (Non-Patent Document 1). An infection in the uterine cervix is called cervical inflammation. On the other hand, an inflammation in the uterine body is called endometritis for inflammation of the endometrium, myometritis for inflammation of the myometrium, and perimetritis for the inflammation of the chorionic membrane, depending on the affected lesion; however, most of the inflammations are endometritis. The majority of endometritis is ascending infection, and phlogogenic bacteria include Streptococci, Staphylococci, Escherichia coli, anaerobes, and the like. When these bacteria infect villi or amniotic fluid during pregnancy, the bacteria cause chorioamnionitis and premature birth. Since such infections are thought to be caused by a mild disturbance in the intrauterine bacterial flora, improvement of the intrauterine bacterial flora is important.

However, it is the current situation that various measures such as vaginal lavage, antibiotic administration, and hormone therapy as in conventional cases do not exhibit sufficient effects for improving the intrauterine environment. Penicillin antibiotics such as ampicillin and amoxicillin, which are representative antibiotics, have a weak effect on anaerobic bacteria and are not recommended as a therapeutic method for bacterial vaginosis in JAID/JSC Guidelines for Infection Treatment 2014, Sanford. Therefore, it is considered that these antibiotics alone have a low effect of increasing Lactobacillus bacteria. Furthermore, it is also known that disorder occurs in the skin, the liver, and the like as a side effect caused by antibacterial drug treatment although it is rare.

As such, there is an increasing need for the development of therapeutic methods based on normalization of a disturbance in the intrauterine bacterial flora; however, there has been hitherto no report on the methods for treatment and intervention to improve the intrauterine bacterial flora.

Lactoferrin is an iron-binding protein discovered in milk in 1939, and since then, it was found that lactoferrin is included in the milk of many mammals other than cows. Lactoferrin is an iron-binding bioactive protein that is abundant in human breast milk, especially in the colostrum, but is also included in tears, saliva, pancreatic juice, and other exocrine fluids, or neutrophils in adults. Lactoferrin has bacteriostatic activity due to its ability to deprive iron, which is essential for the survival and growth of bacteria, and has antibacterial activity such as bacteriolysis through binding to lipopolysaccharides of the bacterial membrane, inhibition of bacterial adhesion to epithelial cells, and inhibition of invasion into host cells (Non-Patent Document 7).

In 2012, Giunta et al. reported that when 200 mg per day of lactoferrin was orally administered to pregnant women with iron deficiency for one month, vaginal infection was cured, the vaginal microbial flora was normalized, and the risk of premature birth was reduced (Non-Patent Document 8). Similarly, in 2014, Otsuki et al. reported that lactoferrin, which is one of the milk-derived protein components, is effective for the prevention of premature birth (Non-Patent Document 9). According to an article by Otsuki et al. in 2017, it was reported that the vaginal bacterial flora of female patients with refractory bacterial vaginosis who suffered from recurrent premature birth was such that bacteria of the genus Lactobacillus, which is a kind of lactic acid bacterium that is abundant in the normal vagina, was noticeably reduced, the bacteria of the genus Lactobacillus were increased by administering lactoferrin orally (700 mg/day) and directly intravaginally for a long time period, and the female patients finally became pregnant and then gave birth without premature birth (Non-Patent Document 10). In 2017, Pino et al. in Italy reported that when 100 to 200 mg of lactoferrin was intravaginally administered to patients with bacterial vaginosis, and the patients were subjected to a bacterial test after two weeks, Gardnerella, Prevotella, and Lachnospira were noticeably decreased, while Lactobacillus was increased (Non-Patent Document 11). From the above-described results, lactoferrin was acknowledged to have prebiotic action, by which a proliferative effect is exhibited on Lactobacillus bacteria in the vagina, and it was found that lactoferrin was also effective for treating bacterial vaginosis.

As such, with regard to lactoferrin, which is a bioactive protein, the effect of improving the intravaginal bacterial flora has been reported one after another in recent years; however, the effect focused on the intrauterine bacterial flora has not yet been known.

### PRIOR ART

### NON-PATENT LITERATURE

Non-patent literature 1: Baker JM et al., Front Immunol. 2018 Mar 2;9:208. Uterine Microbiota: Residents, Tourists, or invaders?
Non-patent literature 2: Franasiak JM et al., J Assist Reprod Genet. 2016 Jan;33(1):129-36. Endometrial microbiome at the time of embryo transfer: next-generation sequencing of the 16S ribosomal subunit.
Non-patent literature 3: Mitchell CM et al., Am J Obstet Gynecol. 2015 May;212(5):611.e1-9. Colonization of the upper genital tract by vaginal bacterial species in nonpregnant women.
Non-patent literature 4: Moreno I et al., Am J Obstet Gynecol. 2016 Dec;215(6):684-703. Evidence that the endometrial microbiota has an effect on implantation success or failure.
Non-patent literature 5: Chen C et al., Nat Commun. 2017 Oct 17;8(1):875. The microbiota continuum along the female reproductive tract and its relation to uterine-related diseases.
Non-patent literature 6: Kyono K et al., Reprod Med Biol. Jul; 17(3): 297-306. Analysis of endometrial microbiota by 16S ribosomal RNA gene sequencing among infertile patients: a single-center pilot study.
Non-patent literature 7: Valenti P et al. Front Immunol. 2018; 9: 376. Role of Lactobacilli and Lactoferrin in the Mucosal Cervicovaginal Defense.
Non-patent literature 8: Giunta G et al., Mol Med Rep. 2012 Jan;5(1):162-6. Influence of lactoferrin in preventing preterm delivery: a pilot study.
Non-patent literature 9: Otsuki et al., J Obstet Gynaecol Res. 2014 Feb;40(2):583-5. Administration of oral and vaginal prebiotic lactoferrin for a woman with a refractory vaginitis recurring preterm delivery: appearance of lactobacillus in vaginal flora followed by term delivery.
Non-patent literature 10: Otsuki et al., Biochem Cell Biol. 2017 Feb;95(1):31-33. Effects of lactoferrin in 6 patients with refractory bacterial vaginosis.
Non-patent literature 11: Pino A et al., Microb Ecol Health Dis. 2017; 28(1): 1357417. Bacterial biota of women with bacterial vaginosis treated with lactoferrin: an open prospective randomized trial.
Non-patent literature 12: Walters, W. et al., mSystems, 2016; 1(1), e00009-15. Improved bacterial 16S rRNA gene (V4 and V4-5) and fungal internal transcribed spacer marker gene primers for microbial community surveys.

### SUMMARY OF THE INVENTION

### PROBLEM TO BE SOLVED BY THE INVENTION

An object of the present invention is to provide an agent and a composition for improving the intrauterine bacterial flora. Another object of the invention is to provide a method for determining whether the intrauterine bacterial flora has been improved or normalized.

### MEANS FOR SOLVING PROBLEM

The inventors of the present invention found that lactoferrin improves the intrauterine bacterial flora and thus completed the present invention.

According to the invention, the following are provided:
[1] an agent for improving bacterial flora in the uterus, comprising lactoferrin or a salt thereof as an active ingredient;
[2] a therapeutic or prophylactic agent for a disease caused by a disturbance in the intrauterine bacterial flora, such as infertility, miscarriage, premature birth, recurrent pregnancy loss, endometriosis, endometrial cancer, cervicitis, endometritis, and/or chorioamnionitis, comprising lactoferrin or a salt thereof as an active ingredient;
[3] a composition comprising the agent according to the above-described [1] or [2];
[4] the composition according to the above-described [3], which is a pharmaceutical composition;
[5] the composition according to the above-described [3], which is a food or drink composition;
[6] a method for determining whether the intrauterine bacterial flora has been improved or normalized, comprising a step of measuring the Lactobacillus occupancy rate in the intrauterine bacterial flora by a bacterial flora analysis technique based on the amplification of the 16S ribosome RNA gene using a plurality of specimens collected from the inside of the uterus of a subject at two or more time points selected from before the administration or ingestion, during the administration or ingestion, and after the administration or ingestion, of the agent or composition according to any one of the above-described [1] to [5]; and a step of calculating the differences between the occupancy rates of the plurality of specimens;
[7] the method according to the above-described [6], wherein the bacterial flora analysis technique is an amplicon sequencing method of using the 16S ribosome V4 region as an amplification target;
[8] a method for treating or preventing a disease caused by a disturbance in the intrauterine bacterial flora, comprising administering an agent for improving the intrauterine bacterial flora comprising lactoferrin or a salt thereof as an active ingredient, or a composition comprising said agent to a subject; and
[9] the method according to the above-described [8], wherein the disease caused by a disturbance in the intrauterine bacterial flora is any one or more of infertility, miscarriage, premature birth, recurrent pregnancy loss, endometriosis, endometrial cancer, cervicitis, endometritis, and/or chorioamnionitis.

### EFFECT OF THE INVENTION

According to the present invention, an effective means for normalizing the intrauterine bacterial flora is provided. Lactoferrin is a component that is also included in breast milk and is very highly safe, and since there is almost no risk of side effects, it is also easy to use lactoferrin in combination with other medicines. Furthermore, since lactoferrin has also been utilized as an ingredient of foods and supplements and can be easily administered orally or ingested, the intrauterine bacterial flora can be improved without requiring a surgical operation by a hospital. Therefore, the intrauterine bacterial flora can be safely and conveniently normalized by the agent or composition of the present invention.

The effect of the agent or composition of the present invention can be conveniently determined by the method of the present invention for determining whether the intrauterine bacterial flora has been improved or normalized, and the determination of the necessity of continuation of the administration or ingestion of the agent or composition of the present invention as well as the necessity of addition or modification of other treatments can be easily carried out.

### MODE(S) FOR CARRYING OUT THE INVENTION

The agent of the present invention comprises lactoferrin or a salt thereof as an active ingredient. Furthermore, the composition of the present invention, for example, a pharmaceutical composition or a food or drink composition, is such a composition comprising the agent of the present invention comprising lactoferrin as an active ingredient. The agent or the composition of the present invention, for example, a pharmaceutical composition or a food or drink composition, is effective for an improvement of the intrauterine bacterial flora. The improvement of the intrauterine bacterial flora according to the present invention implies increasing of the Lactobacillus occupancy rate in the intrauterine bacterial flora. The method for determining improvement or normalization of the intrauterine bacterial flora will be described below.

Lactoferrin is a macromolecule having a molecular weight of about 80,000 and has a property of forming a chelate with two trivalent iron ions; however, the "lactoferrin" as used in the present invention is not limited to biologically derived lactoferrin extracted from the milk and the like of mammals, and any lactoferrin may be used as long as it exhibits the biological activity of lactoferrin, particularly the action of improving the endometrial bacterial flora. Examples thereof include naturally occurring lactoferrins (for example, bovine lactoferrin included in bovine milk) obtainable from various mammals including human being (for example, cow, horse, pig, sheep, goat, and camel etc.), apolactoferrin (iron ion-free type) obtained by removing iron from lactoferrin by a routine method, metal-saturated or unsaturated lactoferrin obtained by chelating metal (iron, copper, zinc, manganese, or the like) ions with apolactoferrin, genetically recombined lactoferrins produced by genetic engineering technology, and products obtained by conjugating polyethylene glycol chains to these lactoferrins. In addition, genetically recombined lactoferrins include a recombinant lactoferrin produced based on modified lactoferrin gene, as well as lactoferrin secreted by transgenic animals, functional equivalents of active fragments of lactoferrin, and the like.

A salt of lactoferrin that can be used for the present invention is a physiologically acceptable salt of any arbitrary lactoferrin such as described above, and examples include sodium salt, potassium salt, sulfuric acid salt, and phosphoric acid salt.

The agent or composition of the present invention may comprise only one kind of lactoferrin such as described above or may comprise two or more kinds thereof. Since lactoferrin is a known substance, a commercially available product can be used. Furthermore, a lactoferrin purified from milk containing lactoferrin or the like by a known method, for example, a method for purifying lactoferrin using a sulfonated carrier (JP H3-109400 A), can be used. In addition, depending on the use application, a fraction from milk or the like, which contains lactoferrin at a high concentration (for example, a fraction obtained by removing saccharides from milk), can also be used.

The agent of the present invention comprises lactoferrin as the only essential component; however, the agent or composition of the present invention may comprise various components and additives that are known in the pharmaceutical or food industries, as desired. The form of the pharmaceutical composition and the food or drink composition of the present invention is not particularly limited. The food or drink composition of the present invention is to be orally administered, and the form of the composition may be various forms such as foods, food materials, beverages, and drinks. The composition of the present invention is preferably in the form of a dosage form convenient for oral administration, such as a powder preparation, a powdered drug, a granular preparation, a tablet, or a capsule.

Examples of additives that may be contained in a composition such as the pharmaceutical composition or the food or drink composition of the present invention include an excipient, a disintegrant, a lubricating agent, a binding agent, a surfactant, a fluidity promoter, a colorant, and a fragrance, which are conventionally used in pharmaceutical or food industries. These additives are appropriately selected according to the desired dosage form or the like.

For example, in a case in which the pharmaceutical composition or the food or drink composition of the present invention is in the form of a powdered preparation, a granular preparation, a tablet, a capsule, or the like, examples of the excipient used include monosaccharides and disaccharides such as lactose, sucrose, glucose, sorbitol, and lactitol; starches such as corn starch and potato starch; crystalline cellulose; and inorganic substances such as light silica gel, synthetic aluminum silicate, magnesium metasilicate aluminate, calcium hydrogen phosphate, and silicon dioxide. Furthermore, in addition to the excipient, a binding agent, a disintegrant, a surfactant, a lubricating agent, a fluidity promoter, a colorant, a fragrance, and the like can be appropriately used as necessary.

Examples of the disintegrant include starches, carboxymethyl cellulose (CMC), hydroxypropyl cellulose (HPC), carboxymethyl cellulose sodium salt, and polyvinylpyrrolidone. Regarding the lubricating agent, sucrose fatty acid esters, calcium stearate, magnesium stearate, and the like can be used.

Examples of the binding agent include starch, dextrin, powdered gum arabic, gelatin, hydroxypropyl starch, sodium carboxymethyl cellulose, methyl cellulose, crystalline cellulose, ethyl cellulose, and polyvinylpyrrolidone.

Examples of the surfactant include soybean lecithin and sucrose fatty acid esters; examples of the lubricating agent include talc, wax, sucrose fatty acid esters, hydrogenated vegetable oils, calcium stearate, and magnesium stearate; and examples of the fluidity promoter include anhydrous silicic acid, dry aluminum hydroxide, and magnesium silicate.

Since lactoferrin is unstable under a high-temperature and highly humid environment, it is preferable that the composition of the present invention is formulated in a dry state.

Since a lactoferrin powder usually has a very low specific gravity and cannot be directly tableted, in order to obtain the composition of the present invention as a preparation that is more stable and has a well-maintained a pharmacological effect, for example, the active ingredient is mixed with an excipient, a binding agent, and a disintegrant, the mixture is compression-molded with a slug machine to produce a large thin flat disc, the disc is crushed and sieved, and granules having a certain size are obtained. In the case of producing tablets, a lubricating agent is added to the granules, the mixture is tableted, and tablets may be covered with a coating film as desired to be manufactured as commercial products. Furthermore, in the case of producing capsules, a capsule may be filled with a certain amount of the granules to be produced as capsules.

The oral (pharmaceutical or food and drink) composition of the present invention may also be produced as an enteric-coated preparation. It is known that lactoferrin is decomposed by pepsin, which is a proteolytic enzyme in the stomach, in an acidic state with gastric acid, and it is considered that the absorption action site of lactoferrin exists in the intestinal tract centered on the small intestine. The method for processing lactoferrin into an enteric-coated preparation is not particularly limited. For example, in order to prepare an enteric-coated preparation, an enteric-coated capsule may be prepared with a coating film containing a base that is resistant to gastric fluid and dissolves in the small intestine, for example, a base selected from the group consisting of shellac, hydroxypropyl methylcellulose phthalate, carboxymethyl ethyl cellulose, cellulose acetate phthalate, a methacrylic acid copolymer, water-insoluble ethyl cellulose, and an aminoalkyl methacrylate copolymer, as a main component, and the capsule may be filled with granules comprising an active ingredient, or a lubricating agent may be added to granules comprising an active ingredient, and the mixture may be tableted, and the tablet thus obtained may be coated with the above-described coating film.

Whether the prepared composition is enteric-coated can be checked by testing the disintegrability using the liquid No.1 (pH 1.2, Japanese Pharmacopoeia, General Testing Methods 41) prepared by adding hydrochloric acid and water to 2.0 g of sodium chloride, dissolving the salt, and making the solution up to 1000 ml, and the liquid No.2 (pH 6.8) prepared by adding 118 ml of 0.2 N sodium hydroxide test solution and water to 250 ml of a 0.2 M potassium dihydrogen phosphate test solution up to 1000 ml. Tablets or granules that do not disintegrate when immersed in the liquid No.1 for 120 minutes and disintegrate when immersed in the liquid No.2 for 60 minutes, do not dissolve in the stomach but start to disintegrate only after flowing into the duodenum, so that the active ingredient is eluted, and the tablets or granules can be considered to be enteric-coated.

Regarding a method capable of delivering lactoferrin to the intestinal tract other than the use of an enteric-coated preparation, the use of a liposome preparation composed of a lipid bilayer is mentioned. A liposome preparation also does not disintegrate in the stomach but is emulsified by bile juice and disintegrates in the small intestine, and therefore, a liposome preparation can deliver lactoferrin to the absorption site in the intestinal tract. Alternatively, a method of simply increasing the pH in the stomach, that is using an alkali agent in combination, to prevent pepsin from acting on lactoferrin, may also be used.

Examples of the route of administration of the agent or composition of the present invention include oral, transdermal, injection, transintestinal, and intrarectal administration. A preferred route of administration for the pharmaceutical composition and the food or drink composition of the present invention is the oral route.

The agent or composition of the present invention may be administered alone or may be used in combination with other agent. Furthermore, in a case in which the agent or composition of the present invention is used in combination with other agent, the two may be used simultaneously or may be used one after the other.

The amount of administration or ingestion per day of the agent or composition of the present invention effective for improving the intrauterine bacterial flora varies depending on the preparation form, the method of administration, the age and body weight of the subject, and the like; however, in the human being, generally, about 0.1 mg to about 5,000 mg, preferably about 0.5 mg to about 2,000 mg, and most preferably about 10 mg to about 1,000 mg, as lactoferrin per day as an active ingredient can be administered all at once or in divisions, before a meal, after a meal, between meals, and/or before bedtime, or the like.

The agent or composition of the present invention can be administered to human beings and non-human animals, and preferably mammals. Examples of the non-human animals include domestic animals such as cow, horse, pig, and sheep, and companion animals such as dog and cat. The amount of administration or ingestion in a mammal such as a pet (excluding human being) is preferably 0.2 mg to 300 mg/kg of body weight/day as lactoferrin.

Whether the intrauterine bacterial flora has been improved or normalized by the agent or composition of the present invention can be determined by measuring the Lactobacillus occupancy rate in the intrauterine bacterial flora by a bacterial flora analysis technique based on the amplification of the 16S rRNA gene using a specimen collected from the uterus of a test subject at two or more time points selected from before administration or ingestion, during administration or ingestion, or after administration or ingestion of the agent or composition of the present invention, comparing the occupancy rates of a plurality of specimens collected at different time points of collection, and calculating the differences.

The specimen is a sample collected from the inside of the uterus and may be a sample including the intrauterine bacteria, and the method of collection is also not limited; however, for example, the uterine cavity fluid collected using a suction pipette, or the like is used.

The combination of the time point of collecting specimens is not particularly limited and is acceptable so long as there is an interval of one day or longer between the time points, and the time points may be any time points before administration or ingestion, during administration or ingestion, or after administration or ingestion of the agent or composition of the present invention. It is preferable that the specimens obtained at two or more time points include at least one specimen obtained during administration or ingestion, or after administration or ingestion of the agent or composition of the present invention. For example, in the case of collecting specimens at two time points, it is preferable to collect at any combination of before administration or ingestion and during or after administration or ingestion of the agent or composition of the present invention; two different time points during administration or ingestion; during administration or ingestion and after administration or ingestion; and two different time points after administration or ingestion.

The bacterial flora analysis technique used for the determination method of the present invention generally comprises the following steps: (1) the genomic DNA in a specimen collected from a test subject is extracted, and partial or full lengths of the DNA sequences of bacteria-derived 16S rRNA gene is amplified; (2) a library in which an adapter sequence is added to the DNA amplification products is produced, and sequencing is performed with a next-generation sequencer; and (3) the sequence reads thus obtained are combined with a data analysis software, and the origin of bacteria and the occupancy rates in the specimen are estimated.

Regarding the bacterial flora analysis technique based on the amplification of the 16S rRNA gene, for example, a 16S amplicon sequencing method, a shotgun sequencing method, a full-length 16S amplicon sequencing method, or any bacterial flora analysis methods equivalent to those techniques is mentioned. Regarding the amplification target region of the 16S rRNA gene, it is preferable to use the variable regions of V1-V2, V3-V5, and/or V4, which are generally used for 16S rRNA sequencing of human specimens. Particularly, it is preferable to use a primer set intended for the V4 region, with which the bacteria existing in the genital organs can be detected with high sensitivity, and amplicon sequencing methods intended for the V4 region are most preferred because a wide range of microorganisms including Pseudomonas, Escherichia, Salmonella, Lactobacillus, Enterococcus, Listeria, Bacillus, Gardnerella, Bifidobacterium, and two kinds of yeast can be detected.

According to the present invention, the state in which the intrauterine bacterial flora has been normalized refers to a state in which when a specimen collected from the inside of the uterus is measured by a bacterial flora analysis technique based on amplification of the 16S rRNA gene as described above, the Lactobacillus occupancy rate is 50% or higher, and this Lactobacillus occupancy rate is preferably 70% or higher, and more preferably 90% or higher. In a case in which the difference between measured values obtained by various methods for the same specimen exceeds the general range of error, the value measured by a bacterial flora analysis method according to the 16S amplicon sequencing method of using a next-generation sequencer (for example, trade name "MiSeq" (Illumina K.K.)) is used as a reference.

According to the present invention, the state in which the intrauterine bacterial flora has been improved implies that in a case in which specimens collected from the inside of the uterus at two time points are measured by the bacterial flora analysis method as described above, the Lactobacillus occupancy rate in the specimen collected later has increased as compared to the specimen collected earlier.

### EXAMPLES

Hereinafter, the present invention will be described more specifically by way of Examples.

### [Example 1]

The inventors of the present invention conducted an intrauterine bacterial flora analysis on *in vitro* fertilization patients of age 45 or less who consulted an infertility treatment clinic, and as a result, patients (test subjects) whose intrauterine bacterial balance was disturbed were subjected to oral administration of lactoferrin to examine the effect.

### <Intrauterine bacterial flora analysis>

The intrauterine bacterial flora analysis was carried out as follows, basically according to the method described in Non-Patent Document 6. The mucus of the cervical canal and the uterine cervix of a test subject was wiped off, a catheter for embryo transplantation (trade name "Kitazato (registered trademark) IUI catheter" (Kitazato Corporation, Japan)) was carefully inserted through the vagina into the uterus, and the uterine cavity fluid was collected as a specimen. The collected uterine cavity fluid was transferred into 1 mL of a preservative solution for bacterial inactivation and stabilization (trade name "MMB collection tube" (DNA Genotek Inc., Canada)).

In order to extract the bacterial genomic DNA, Proteinase K and lysozyme were added to the preservative solution to dissolve bacteria. The genomic DNA was extracted using a DNA extraction kit (trade name "Agencourt Genfind v2 Blood & Serum DNA Isolation Kit" (Beckman Coulter, Inc., USA)). The concentration of the extracted DNA was measured using trade name "Qubit dsDNA HS Assay Kit" (Thermo Fisher Scientific K.K.).

The bacterial flora analysis was carried out according to the 16S amplicon sequencing method of using a next-generation sequencer. Based on the protocol of "Earth Microbiome Project" (Non-Patent Document 12), amplification of bacterial DNA was carried out using a primer in which a sequence that amplifies the V4 region of the 16S rRNA gene and an Illumina Nextera XT adapter sequence were linked (Non-Patent Document 6). A mixed liquid of 25 ng/ µL of DNA for PCR, 200 µmol/L of deoxyribonucleotide triphosphate, 400 nmol/L of each primer, 2.5 U of trade name "FastStart HiFi polymerase", 20 mg/mL of BSA (Sigma), 0.5 mol/L of betaine (Sigma), and a buffer including MgCl₂ (Roche) was produced. PCR was performed with a thermal cycler (trade name "SimpliAmp Thermal Cycler" (Thermo Fisher)), and the DNA was denatured at 94°C for 2 minutes, subsequently subjected to 30 cycles, each cycle including maintaining for 20 seconds at 94°C, 30 seconds at 50°C, and 1 minute at 72°C, and finally the DNA was reacted for 5 minutes at 72°C. The PCR product was purified with trade name "Agencourt AMPure XP" (Beckman Coulter, Inc., USA). Next, based on "Illumina 16S Metagenomic Sequencing Library Preparation protocol" (https://support.illumina.com/documents/documentation/chemi stry_documentation/16s/16s-metagenomic-library-prep-guide-15044223-b.pdf), a library was created using trade name "Nextera XT Index kit" (Illumina, Inc., USA). For the library thus created, sequencing was performed by pair-end sequencing of 2×200-bp using trade name "MiSeq Reagent Kit v3" (Illumina K.K.). Regarding the data analysis, a quality check of the entire sequence was performed using "fastqQC" (https://www.bioinformatics.babraham.ac.uk/projects/fastqc/ ), and identification of bacteria to the genus level was performed using "USEARCH" (https://www.drive5.com/usearch/) and "QIIME" (http://qiime.org/).

### <Case 1>

In this test subject, a non-Lactobacillus Dominant Microbiota composed of 40% of Lactobacillus bacteria and 60% of Streptococcus bacteria was recognized. This test subject was administered with 750 mg/day of amoxicillin, a penicillin antibiotic, for 7 days. Subsequently, a commercially available Lactoferrin preparation (trade name "LACTOFERRIN GX" (manufactured by NRL Pharma, Inc.)) was orally administered at a dose of 300 mg/day as the amount of lactoferrin. After 50 days of continuous lactoferrin administration, a second intrauterine bacterial flora analysis was performed, and as a result, Lactobacillus bacteria increased to 100%, and an improvement of the intrauterine bacterial flora was recognized.

Amoxicillin has a weak effect on anaerobic bacteria, amoxicillin alone has a low effect of increasing Lactobacillus bacteria, and rather, there is a possibility that Lactobacillus bacteria may be a target of amoxicillin. Therefore, it is considered that by the administration of lactoferrin, Lactobacillus bacteria proliferated in the uterus, and the intrauterine bacterial flora was improved.

### <Case 2>

In this test subject, the intrauterine bacterial flora was extremely unbalanced, and the results of the initial intrauterine bacterial flora analysis were 0.1% of Lactobacillus bacteria, 94.7% of Streptococcus bacteria, and 5.2% of others. This test subject was administered with 750 mg/day of amoxicillin for 14 days. From the 5th day after the initiation of administration of amoxicillin (32^{nd} day after the initial examination), the same lactoferrin preparation as that of Case 1 was continuously orally administered at a dose of 300 mg/day as the amount of lactoferrin. In the second intrauterine bacterial flora analysis carried out after completion of the administration of amoxicillin (57^{th} day after the initial examination/25^{th} day after the initiation of administration of lactoferrin), Streptococci were reduced to 17%, and Lactobacilli were increased to 16.2%; however, a noticeable increase in other bacteria such as Escherichia bacteria was recognized. Since the effect of amoxicillin alone was not recognized, 300 mg/day of cefdinir, a cephem antibiotic, was administered for 7 days from the 53^{rd} day after the initiation of administration of lactoferrin (85^{th} day after the initial examination), and also, a probiotic tampon (trade name "Florgynal Tampon Probiotique" (Laboratoires IPRAD, Paris, France)) was used in combination. After completion of these treatments, only the administration of the lactoferrin preparation was continued, and as a result, the proportion of Lactobacillus bacteria was improved to 98.1% in the third examination carried out on the 119^{th} day from the initial examination. The administration of the lactoferrin preparation was continued for 142 days at a dose of 300 mg/day as the amount of lactoferrin. This test subject subsequently naturally conceived.

Therefore, it was revealed that lactoferrin can improve an imbalance of the intrauterine bacterial flora and can exhibit an effect of preventing or improving various problems caused by a disturbance in the intrauterine bacterial flora. Furthermore, during the implementation of this test, there were no reports of side effects. Therefore, it can be said that this method and the lactoferrin preparation used therefor are highly safe.

### [Example 2] Production of lactoferrin tablets

To 20 kg of raw lactoferrin powder extracted from milk (purity of 95% or higher as a protein; lactoferrin in proteins was 90% or more), 18.5 kg of lactose, 19.7 kg of crystalline cellulose (trade name "Avicel"), 1.2 kg of carboxymethyl cellulose calcium salt, and 0.6 kg of sucrose fatty acid esters were added, a mixture thus obtained was pulverized with a mixer, and a powder that passed through 100-mesh sieve was obtained. This mixed powder was tableted using a tableting machine to obtain tablets each having a major axis of 9 mm and a weight of 300 mg. One tablet contained 100 mg of the raw lactoferrin powder.

### [Example 3] Production of enteric-coated lactoferrin tablets (part 1)

The tablets produced in Example 1 were introduced into a coating machine (manufactured by Freund Corporation, HICOATER HCT-48N). An enteric coating liquid composed of 9.6 wt%of shellac, 1.5 wt% of L-arginine, 1.9 wt% of sorbitol, 2.4 wt% of sucrose fatty acid esters, 4.8 wt% of ethanol, and 79.8% of purified water was sprayed on those tablets, and an enteric coating was provided at a ratio of 8% to 9% by mass with respect to the tablets to obtain a manufactured product.

### [Example 4] Production of enteric-coated lactoferrin tablets (part 2)

The tablets produced in Example 1 were introduced into a coating machine (manufactured by Freund Corporation, HICOATER HCT-48N). An enteric coating liquid composed of 9 wt% of carboxymethyl cellulose, 1 wt% of glycerin fatty acid esters, 45 wt% of ethanol, and 45 wt% of methylene chloride was sprayed on those tablets, and an enteric coating was provided at a ratio of 12% by mass with respect to the tablets to obtain a manufactured product.

### [Example 5] Production of enteric-coated lactoferrin tablets (part 3)

The tablets produced in Example 1 were introduced into a coating machine (manufactured by Freund Corporation, HICOATER HCT-48N). An enteric coating liquid obtained by dissolving 8 parts by mass of zein, which is a protein obtainable from corn kernel, and 2 parts by mass of glycerin in 150 parts by mass of a 70 wt% aqueous solution of ethanol was sprayed on those tablets, and tablets with a coating at a ratio of 10% by mass with respect to the tablets were obtained.

### [Example 6] Production of enteric-coated lactoferrin tablets (part 4)

The tablets produced in Example 1 were introduced into a coating machine (manufactured by Freund Corporation, HICOATER HCT-48N). An enteric coating liquid obtained by dissolving 30 parts by mass of shellac and 7 parts by mass of castor oil in 63 parts by mass of isopropanol was sprayed on those tablets, and tablets with a coating at a ratio of 10% by mass with respect to the tablets were obtained.

This patent application is based on Japanese Patent Application No. 2018-176473, filed on September 20, 2018, and the subject matters described in the specification and the scope of the claims of Japanese Patent Application No. 2018-176473 are all incorporated in this specification.

## Claims

1. An agent for improving bacterial flora in the uterus, comprising lactoferrin or a salt thereof as an active ingredient.

2. A therapeutic or prophylactic agent for a disease caused by a disturbance in the intrauterine bacterial flora, such as infertility, miscarriage, premature birth, recurrent pregnancy loss, endometriosis, endometrial cancer, cervicitis, endometritis, and/or chorioamnionitis, comprising lactoferrin or a salt thereof as an active ingredient.

3. A composition comprising the agent according to the above-described claim 1 or 2.

4. The composition according to the above-described claim 3, which is a pharmaceutical composition.

5. The composition according to the above-described claim 3, which is a food or drink composition.

6. A method for determining whether the intrauterine bacterial flora has been improved or normalized, comprising a step of measuring the Lactobacillus occupancy rate in the intrauterine bacterial flora by a bacterial flora analysis technique based on the amplification of the 16S ribosome RNA gene using a plurality of specimens collected from the inside of the uterus of a subject at two or more time points selected from before the administration or ingestion, during the administration or ingestion, and after the administration or ingestion, of the agent or composition according to any one of the above-described claims [1] to [5]; and a step of calculating the differences between the occupancy rates of the plurality of specimens.

7. The method according to the above-described claim 6, wherein the bacterial flora analysis technique is an amplicon sequencing method of using the 16S ribosome V4 region as an amplification target.

8. A method for treating or preventing a disease caused by a disturbance in the intrauterine bacterial flora, comprising administering an agent for improving the intrauterine bacterial flora comprising lactoferrin or a salt thereof as an active ingredient, or a composition comprising said agent to a subject.

9. The method according to the above-described claim 8, wherein the disease caused by a disturbance in the intrauterine bacterial flora is any one or more of infertility, miscarriage, premature birth, recurrent pregnancy loss, endometriosis, endometrial cancer, cervicitis, endometritis, and/or chorioamnionitis.
